# EUROPEAN PATENT SPECIFICATION

(11) **EP 4 566 555 B1**
(45) Date of publication and mention of the grant of the patent: **02.09.2026**
(21) Application number: 23859862.7
(22) Date of filing: 14.07.2023
(51) Int. Cl.: A61B 17/72, A61B 17/74

(54) **INTRAMEDULLARY NAIL AND BONE FIXATION INSTRUMENT**
MARKNAGEL UND KNOCHENFIXATIONSINSTRUMENT
CLOU INTRAMÉDULLAIRE ET INSTRUMENT DE FIXATION OSSEUSE

(30) Priority: 29.08.2022 JP 2022135876
(43) Date of publication of application: 11.06.2025
(73) Proprietor: Hoya Technosurgical Corporation, Tokyo 160-0004 (JP)
(72) Inventor: ONOSE, Takeshi, Tokyo 160-0004 (JP); YANAGIDA, Shun, Tokyo 160-0004 (JP)
(74) Representative: VKK Patentanwälte PartG mbB
(86) International application number: PCT/JP2023/025985
(87) International publication number: WO 2024/048092

(56) References cited:
- WO-A1-2016/174720
- WO-A1-2016/174720
- JP-A- 2014 512 853
- US-A1- 2014 088 595
- US-A1- 2018 250 042

## Description

The present invention relates to an intramedullary nail and a bone fixation instrument that are used for bone fixation.

Conventionally, a surgical tool called an intramedullary nail is used to treat a bone fracture in the neighborhood of the bone head of, for example, a femur or a humerus. An intramedullary nail is inserted into the medullary cavity of a bone in the longitudinal direction of the bone. Thereafter, a shaft-like member called a lag screw is inserted through the intramedullary nail and screwed into the bone, so that the intramedullary nail, together with a shaft member, strongly fixes the fractured bone from within the bone.

As described in, for example, Japanese Unexamined Patent Application Publication (Translation of PCT Application) No. 2014-512853, the intramedullary nail is cylindrical because the intramedullary nail is inserted into the bone by first inserting a guide wire into the medullary cavity of the bone and then inserting this guide wire through the intramedullary nail.

WO 2016/174720 A1 discloses a cannulated intramedullary nail comprising: a screw hole configured to receive a lag screw; a through hole formed along the longitudinal axis of the intramedullary nail; a first female-thread region disposed in the through hole proximally of the screw hole, a second female-thread region disposed in the through hole proximally of the first female-thread region, and a non-threaded recess disposed in the through hole between the first female-thread region and the second female-thread; a set screw configured to be screwed into the first female-thread region and to prevent movement and rotation of the lag screw relative to the nail by pressing on the lag screw; a washer configured to be disposed in the non-threaded recess and to prevent fallout of the set screw; and an end cap configured to be threaded into the second female-thread region.

The intramedullary nail that is thus cylindrical internally includes a restriction body called a set screw that is cylindrical likewise. This restriction body is configured to restrict the relative movement and rotation of a shaft-like member (lag screw) to the intramedullary nail.

Unfortunately, with the conventional intramedullary nail described above, there is a possibility that the restriction body (set screw) may be displaced from its prescribed position due to an external factor such as vibration. Such displacement may result in insufficient fixation of the shaft-like member (lag screw) to the intramedullary nail or may cause the restriction body (set screw) to fall off the intramedullary nail during transport. Thus, the conventional intramedullary nail has insufficient usability and needs to be improved in this regard.

Accordingly, the present invention provides an intramedullary nail as defined in claim 1 and a bone fixation

instrument as defined in claim 12 that are improved in usability. Preferred features of the invention are defined in the dependent claims.

An intramedullary nail according to one aspect of the present invention is inserted into a bone during bone fixation and, together with a bone fixation shaft member inserted through the intramedullary nail, fixes the bone from within the bone. The intramedullary nail includes a nail main body, a restriction body, and a ring body. The nail main body is cylindrical, has a shaft-member insertion hole formed at an intermediary position thereof in a longitudinal direction thereof and penetrating an outer circumferential surface thereof, and is configured to have the bone fixation shaft member inserted therethrough, and has a first female-thread region disposed closer to a base end of the nail main body in the longitudinal direction than the shaft-member insertion hole, a second female-thread region disposed closer to the base end than the first female-thread region, and a non-threaded region disposed between the first female-thread region and the second female-thread region in the longitudinal direction formed on an inner circumferential surface thereof. The restriction body is cylindrical and has a restriction-body side male-thread region formed on an outer circumferential surface thereof and screwable into the first female-thread region and the second female-thread region. At a restriction position where the restriction body is screwed into the first female-thread region, the restriction body restricts the relative movement and rotation of the bone fixation shaft member to the nail main body by pressing, toward the tip end, the bone fixation shaft member disposed in the shaft-member insertion hole. The ring body is annular about a first axis line and has a ring-body side male-thread region formed on an outer circumferential surface thereof and screwable into the second female-thread region. The ring body is disposed closer to the base end than the restriction body at the restriction position, at an in-use position and at least partially disposed within the non-threaded region. The first female-thread region and the second female-thread region are formed about a second axis line extending in the longitudinal direction. An area where the ring-body side male-thread region is formed in a direction along the first axis line is smaller than an area where the non-threaded region is formed in a direction along the second axis line.

In the above intramedullary nail, a thickness dimension of the ring body in a direction along the first axis line may be smaller than an area where the non-threaded region is formed in a direction along the second axis line.

In the above intramedullary nail, the non-threaded region may have a section that is circular about the second axis line, and an outer diameter of a thread ridge in the ring-body side male-thread region may be smaller than an inner diameter of the non-threaded region.

In the above intramedullary nail, an inner diameter of a thread ridge in each of the first female-thread region and the second female-thread region may be smaller than an inner diameter of the non-threaded region.

In the above intramedullary nail, the restriction-body side male-thread region may be formed about the third axis line, and an area where the restriction-body side male-thread region is formed in a direction along the third axis line may be larger than an area where the non-threaded region is formed in a direction along the second axis line.

In the above intramedullary nail, an inner circumferential surface of the ring body may include a tapered annular surface formed about the first axis line and having an inner diameter that gradually decreases from one side toward the other side in a direction along the first axis line. The one side is a side facing the base end when the ring body is at the in-use position.

In the above intramedullary nail, the restriction body may have a restriction-body end surface that faces the base end at the restriction position, and the ring body may have a ring-body end surface that faces the tip end at the in-use position and comes face-to-face from the side of the base end with the restriction-body end surface of the restriction body at the restriction position.

In the above intramedullary nail, the restriction body may be cylindrical about a third axis line, and an inner circumferential edge of the restriction-body end surface may be disposed radially outside of an inner circumferential edge of the ring-body end surface when the first axis line and the third axis line are coaxially placed.

In the above intramedullary nail, the restriction-body end surface may have a restriction-body inner hole formed inside thereof that is circular about a third axis line extending in the longitudinal direction when the restriction body is at the restriction position, and the ring-body end surface may have a ring-body inner hole formed inside thereof that is circular about the first axis line and has a smaller diameter than the restriction-body inner hole.

In the above intramedullary nail, and the restriction body and the ring body may be formed of the same metallic material, and the entire region of one of the restriction-body end surface and the ring-body end surface may come face-to-face with a part of the entire region of the other thereof in the longitudinal direction.

In the above intramedullary nail, the ring body has an engagement portion formed on an inner circumferential surface thereof that is dented radially outwardly or projected radially inwardly, and the ring body may be rotatable about the first axis line by use of a tool with the engagement portion engaged with an engagement receiving portion of the tool.

A bone fixation instrument according to one aspect of the present invention includes the above intramedullary nail and a bone fixation shaft member that is inserted through the shaft-member insertion hole in the intramedullary nail.

The intramedullary nail and the bone fixation instrument described above can improve its usability.
Fig. 1 is an overall side view of a bone fixation instrument according to an embodiment of the present invention as seen through a bone of a patient when the bone fixation instrument has been placed inside the bone.
Fig. 2 is a sectional view of a main part of an intramedullary nail in the bone fixation instrument taken in a lengthwise direction of the intramedullary nail as seen from the front side of the body of the patient when the bone fixation instrument is in use.
Fig. 3A is a sectional view of a nail main body of the intramedullary nail taken in the lengthwise direction along the same section as the view of Fig. 2.
Fig. 3B is a sectional view of the nail main body of the intramedullary nail taken in the lengthwise direction as seen from a side 180 degrees circumferentially opposite to Fig. 3A, that is, from the backside of the body of the patient.
Fig. 4A is a side view of a restriction body in the intramedullary nail as seen from the body of the patient when the bone fixation instrument is in use.
Fig. 4B is a side view of the restriction body in the intramedullary nail as seen from a position 90 degrees circumferentially displaced from Fig. 4A.
Fig. 5 is a sectional view of the restriction body and a ring body in the intramedullary nail that have been placed in the nail main body, taken in the lengthwise direction along the same section as the view of Fig. 2.
Fig. 6A is a top view of the ring body of the intramedullary nail as seen from the side of its base end.
Fig. 6B is a view of the ring body of the intramedullary nail as seen in the direction of arrow A in Fig. 6A.
Fig. 7 is a perspective view of a dedicated tool for rotating the ring body in the intramedullary nail.

The following describes embodiments of the present invention in detail with reference to the drawings.

### (Overall configuration)

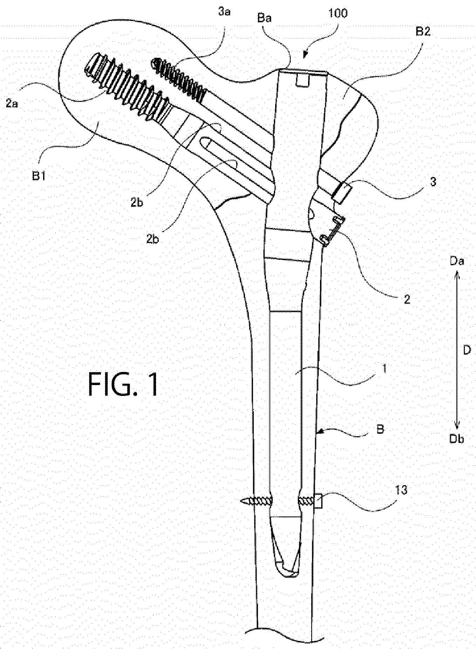

As illustrated in Fig. 1, a bone fixation instrument 100 of the present embodiment is used, for example, in the treatment of a bone fracture (femoral neck fracture) in the neighborhood of the bone head B1 of a femur (hereinafter, simply "bone") B. The bone fixation instrument 100 includes an intramedullary nail 1, a bone fixation shaft member (lag screw) 2, and an auxiliary shaft member 3. The intramedullary nail 1 has a rod-like shape and is inserted into the femur B in a direction in which the femur B extends. The bone fixation shaft member (lag screw) 2 is inserted through the intramedullary nail 1 in a direction intersecting a longitudinal direction D of the intramedullary nail 1. The auxiliary shaft member 3 is provided parallel to the bone fixation shaft member 2 and inserted through the intramedullary nail 1.

### (Intramedullary nail)

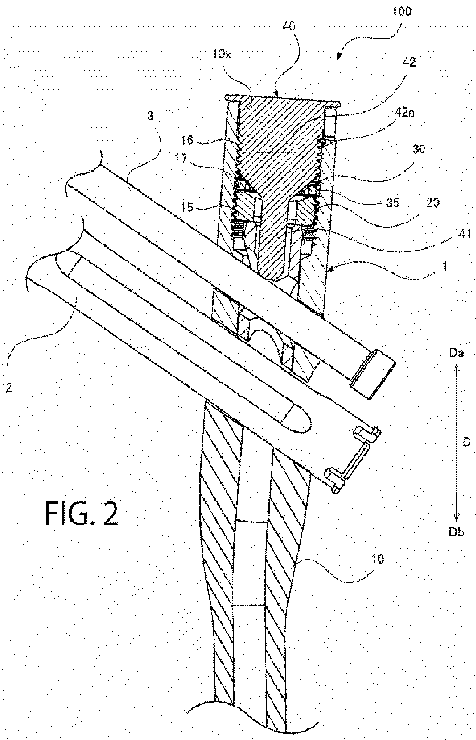

As illustrated in Fig. 2, the intramedullary nail 1 includes a cylindrical nail main body 10, a restriction body 20, a ring body 30, and an end cap 40. The nail main body 10 is inserted into the medullary cavity (lumen) of the femur B so as to extend in a direction in which the femur B extends. The restriction body 20 and the ring body 30 are provided inside the nail main body 10. The end cap 40 blocks an upper opening 10x of the nail main body 10.

### (Nail main body)

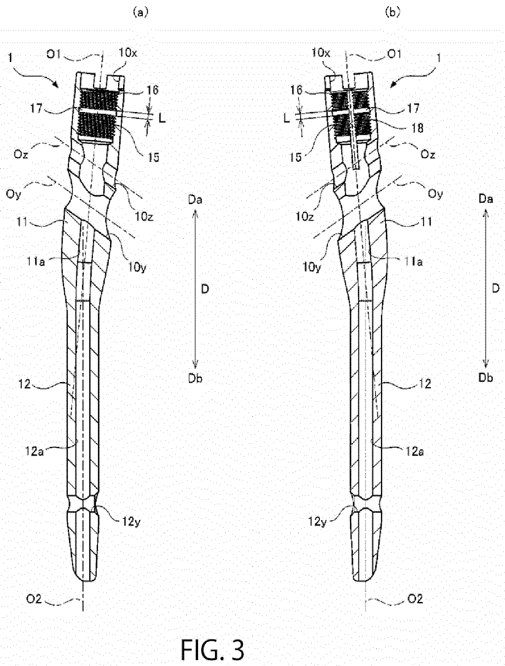

As illustrated in Figs. 3A and 3B, the nail main body 10 includes a proximal portion 11 provided on a side of a base end Da in the longitudinal direction D (the upper side when the bone fixation instrument 100 is in use) and extending in the longitudinal direction D, and a distal portion 12 provided on a side of a tip end Db in the longitudinal direction D to the proximal portion 11 (the lower side when the bone fixation instrument 100 is in use). The distal portion 12 is formed in one body with the proximal portion 11 and extends toward the tip end Db in a bending (or warping) manner from the proximal portion 11.

The proximal portion 11 is shaped in a circular cylinder about a proximal-portion axis line (second axis line) O1 extending in the longitudinal direction D. An inner hole 11a of the proximal portion 11, which is formed by an inner circumferential surface of the proximal portion 11, has a section shaped in a precise circle about the proximal-portion axis line O1. In the neighborhood of an end portion of the proximal portion 11 on the side of the tip end Db connected to the distal portion 12, the diameter of the proximal portion 11 gradually decreases from one side (that faces the base end Da) to the other side (that faces the tip end Db) in the proximal-portion axis line O1.

The distal portion 12 is shaped in a circular cylinder about a distal-portion axis line O2 extending in a direction intersecting the longitudinal direction D and the proximal-portion axis line O1. An inner hole 12a is formed in the distal portion 12, which has a section shaped in a precise circle about the distal-portion axis line O2. As in the case of the proximal portion 11, in the neighborhood of an end portion of the distal portion 12 on the side of the tip end Db, the distal portion 12 has a tapered shape in which the diameter gradually decreases from one side (that faces the base end Da) to the other side (that faces the tip end Db) in the distal-portion axis line O2.

The inner hole 11a of the proximal portion 11 and the inner hole 12a of the distal portion 12 are smoothly connected to each other, so that these inner holes 11a and 12a unitedly penetrate the inside of the nail main body 10 in the longitudinal direction D. These inner holes 11a and 12a function to have therethrough a guide wire (not illustrated), which is inserted into the medullary cavity before the nail main body 10 is inserted into the medullary cavity.

The proximal portion 11 has a shaft-member insertion hole 10y formed therein so as to penetrate through an outer circumferential surface of the proximal portion 11 at an intermediary position of the nail main body 10 in the longitudinal direction D. This shaft-member insertion hole 10y extends upward from the lateral side of the body of the patient toward the midline thereof so that the central axis line Oy thereof may extend in a direction intersecting the proximal-portion axis line O1 and the axis may pass through the trochanter part B2 of a femur B toward the bone head B1 thereof with the bone fixation instrument 100 placed for use (see Fig. 1).

That is, the shaft-member insertion hole 10y communicates with the inner hole 11a of the proximal portion 11 and penetrates an outer circumferential surface of the nail main body 10 at two locations across the inner hole 11a that face toward the lateral side and the midline of the body of the patient with the bone fixation instrument 100 placed for use. This shaft-member insertion hole 10y is configured to allow a bone fixation shaft member 2 (see Fig. 1) to be inserted therethrough, which is described in detail below.

Furthermore, on the side closer to the base end Da than the shaft-member insertion hole 10y, the proximal portion 11 also has an auxiliary shaft-member insertion hole 10z formed so as to penetrate the outer circumferential surface of the proximal portion 11 so that the central axis line Oz of the auxiliary shaft-member insertion hole 10z may extend in parallel to the central axis line Oy of the shaft-member insertion hole 10y. Thus, the auxiliary shaft-member insertion hole 10z communicates with the inner hole 11a of the proximal portion 11 and penetrates the outer circumferential surface of the nail main body 10 at two locations across the inner hole 11a that face toward the lateral side and the midline of the body of the patient with the bone fixation instrument 100 placed for use.

This auxiliary shaft-member insertion hole 10z is configured to allow an auxiliary shaft member 3 to be inserted therethrough, which is described in detail below. The auxiliary shaft member 3 and the bone fixation shaft member 2 that has been inserted through the shaft-member insertion hole 10y are disposed parallel to each other (see Fig. 1) when the auxiliary shaft member 3 is inserted through the auxiliary shaft-member insertion hole 10z.

On the side closer to a tip end Db than the shaft-member insertion hole 10y, the distal portion 12 has a fixation-tool insertion hole 12y formed so as to penetrate an outer circumferential surface of the distal portion 12 in a direction intersecting the longitudinal direction D, that is, in a direction perpendicular to the distal-portion axis line O2. That is, the fixation-tool insertion hole 12y communicates with the inner hole 12a of the distal portion 12 and penetrates the outer circumferential surface of the nail main body 10 at two locations across the inner hole 12a that face toward the lateral side and the midline of the body of the patient with the bone fixation instrument 100 placed for use. A fixation screw 13 for fixing the intramedullary nail 1 to the femur B is inserted into this fixation-tool insertion hole 12y (see Fig. 1).

Furthermore, the inner circumferential surface of the proximal portion 11, that is, a hole inner surface of the inner hole 11a, has a first female-thread region 15, a second female-thread region 16, and a non-threaded region 17 formed thereon on the side closer to the base end Da than the shaft-member insertion hole 10y and the auxiliary shaft-member insertion hole 10z.

The first female-thread region 15 is formed about the proximal portion axis line O1. The first female-thread region 15 is configured to allow the restriction body 20 to be screwed therein, which is described in detail below (see Fig. 2), when the bone fixation instrument 100 is in use.

The second female-thread region 16 is provided on the side closer to the base end Da than the first female-thread region 15 with a distance between the regions in a direction along the proximal-portion axis line O1. The specifications, such as the dimensions and the pitches of the thread ridges and thread grooves in the second female-thread region 16 are the same as those in the first female-thread region 15. The second female-thread region 16 is formed on the side closer to the tip end Db than an open end of the inner hole 11a, which is the end of the nail main body 10 on the side of the base end Da.

The non-threaded region 17 is provided between the first female-thread region 15 and the second female-thread region 16. The non-threaded region 17 is a region on the hole inner surface of the inner hole 11a in the proximal portion 11 on which no female thread is formed, and is formed of a hole having a section shaped in a precise circle about the proximal-portion axis line O1. The non-threaded region 17 has an inner diameter equal to or slightly larger than the inner diameter of a thread groove of each of the first female-thread region 15 and the second female-thread region 16. The non-threaded region 17 defines a space which is larger in diameter than the first female-thread region 15 and the second female-thread region 16.

Furthermore, a guide groove 18 is formed on the hole inner surface of the inner hole 11a. The guide groove 18 opens through the end face of the inner hole 11a of the nail main body 10 on the side of the base end Da, is dented radially outwardly all the way through the first female-thread region 15, the second female-thread region 16, and the non-threaded region 17 in the direction of the proximal-portion axis line O1, and extends beyond the first female-thread region 15 toward the tip end Db. Each of the first female-thread region 15 and the second female-thread region 16 circumferentially is cut off at one position in a circumferential direction thereof by the guide groove 18.

### (Restriction body)

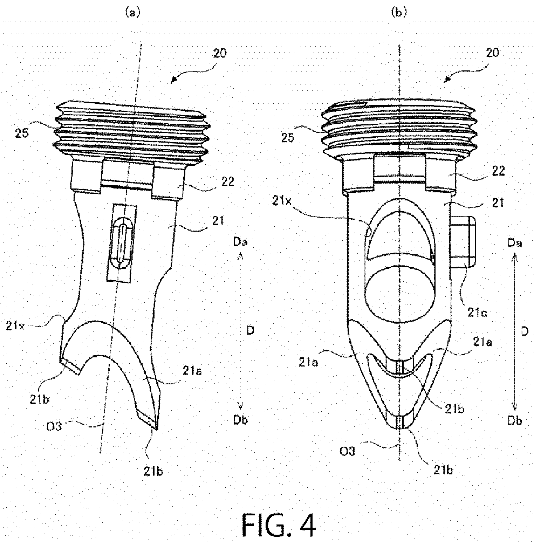

The restriction body 20 is formed of a metallic material such as titanium, as illustrated in Figs. 4A and 4B, and includes a restriction-body main body 21 and a screw portion 22. The restriction-body main body 21 contacts the bone fixation shaft member 2 when the bone fixation instrument 100 is in use. The screw portion 22 rotates relative to the restriction-body main body 21. The restriction body 20 is inserted through the upper opening 10x (see Fig. 2) into the inner hole 11a of the proximal portion 11 of the nail main body 10.

The restriction-body main body 21 is shaped in a circular cylinder about a restriction-body axis line (third axis line) O3 extending in the longitudinal direction D when the bone fixation instrument 100 is in use. The restriction-body main body 21 has a tapered shape in which the outer diameter gradually decreases towards the bottom. Paired sloping flat surfaces 21a are provided at positions 180 degrees circumferentially apart from each other, which are formed in such a manner that an outer circumferential surface is cut out so as to extend radially outwardly toward the base end at a tip end of the restriction-body main body 21 that serves as the lower end thereof when the bone fixation instrument 100 is in use.

Between the sloping flat surfaces 21a, paired pressing portions 21b that are plat-shaped and substantially triangular are formed. These paired pressing portions 21b have tip ends (lower ends) thereof provided at positions shifted in a direction along the restriction-body axis line O3. Each of the paired pressing portions 21b can contact the bone fixation shaft member 2, which is described in detail below, when the bone fixation shaft member 2 is inserted through the shaft-member insertion hole 10y of the nail main body 10 and penetrates the nail main body 10 (see Fig. 2).

A rectangular guide projection 21c is formed on the outer circumferential surface of the restriction-body main body 21 and projects radially outwardly and extends in a direction along the restriction-body axis line O3. When the restriction-body main body 21 is inserted into the inner hole 11a of the nail main body 10, the guide projection 21c is disposed inside the guide groove 18 in the nail main body 10, thereby guiding the restriction-body main body 21 in the longitudinal direction D in the inner hole 11a of the nail main body 10 while restricting the relative rotation of the restriction-body main body 21 to the nail main body 10 (see Fig. 3B).

The restriction-body main body 21 has a restriction-body through-hole 21x formed therein that penetrates the outer circumferential surface thereof in a direction intersecting a direction along the restriction-body axis line O3. This restriction-body through-hole 21x is configured to allow the auxiliary shaft member 3 (see Fig. 2) to be inserted therethrough, which is described in detail below, when the bone fixation instrument 100 is in use. That is, when the bone fixation instrument 100 is in use, the restriction-body through-hole 21x extends upwardly toward the midline of the body of the patient and is placed coaxially with the auxiliary shaft-member insertion hole 10z (see Figs. 3A and 3B) of the nail main body 10.

The screw portion 22 is annular about the restriction-body axis line O3. The screw portion 22 covers the outer circumferential surface of the restriction-body main body 21 on the side of the base end Da of the restriction-body main body 21 to support the restriction-body main body 21. The screw portion 22 can rotate relative to the restriction-body main body 21 about the restriction-body axis line O3. On the outer circumferential surface of the screw portion 22, a restriction-body side male-thread region 25 is formed about the restriction-body axis line O3.

This restriction-body side male-thread region 25 can be screwed into both the first female-thread region 15 and the second female-thread region 16 (see Figs. 3A and 3B) in the nail main body 10. An area where the restriction-body side male-thread region 25 is formed in a direction along the restriction-body axis line O3 is larger than an area where the non-threaded region 17 in the nail main body 10 is formed in a direction along the proximal-portion axis line O1. That is, the restriction-body side male-thread region 25 can be screwed to both the first female-thread region 15 and the second female-thread region 16 while straddling the first female-thread region 15 and the second female-thread region 16.

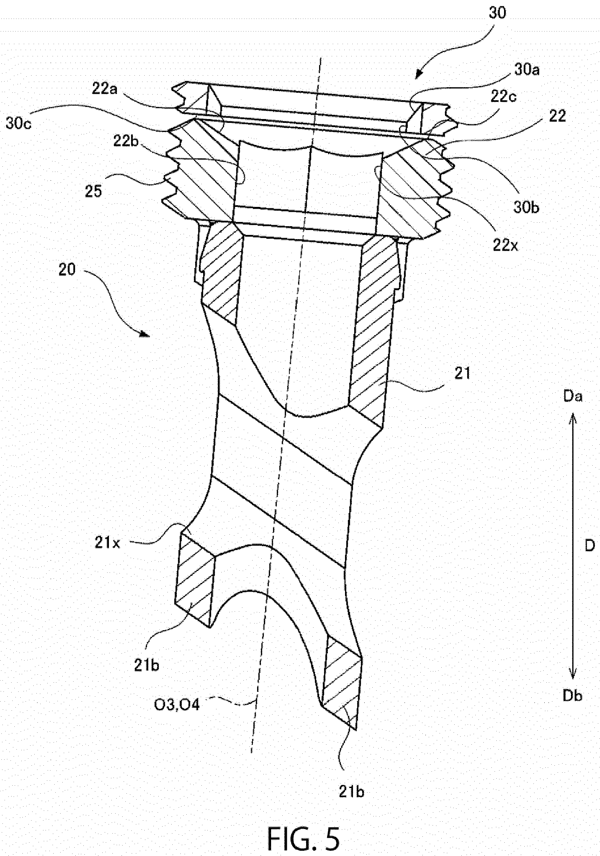

As illustrated in Fig. 5, inside the screw portion 22, a hexagonal hole (or a hole of another type, such as a hexalobular hole) 22x is formed in a region of a restriction-body side straight annular surface 22b, which is described below. The screw portion 22 can be rotated about the restriction-body axis line O3 by using a tool (not illustrated) which engages in this hole 22x. The restriction-body side male-thread region 25 of the screw portion 22 is screwed into the second female-thread region 16 and the first female-thread region 15 (see Figs. 3A and 3B) in this order so that the screw portion 22 is inserted into the inner hole 11a in the nail main body 10. This causes the guide projection 21c of the restriction-body main body 21 to be guided by the guide groove 18 (see Fig. 3B) so that the restriction-body main body 21 is inserted into the nail main body 10 with the screw portion 22 making a relative rotation to the restriction-body main body 21 about the restriction-body axis line O3 and with the relative rotation of the restriction-body main body 21 to the nail main body 10 being restricted.

At a position where the restriction-body side male-thread region 25 of the screw portion 22 has been screwed only with the first female-thread region 15, the pressing portions 21b of the restriction-body main body 21 contact the bone fixation shaft member 2 and press the bone fixation shaft member 2 toward the tip end Db in a direction along the restriction-body axis line O3. Thus, a position of the restriction body 20 where the pressing portions 21b press the bone fixation shaft member 2 serves as a restriction position. When the restriction body 20 is placed at this restriction position, the relative movement and rotation of the bone fixation shaft member 2 to the nail main body 10 are thereby restricted. Furthermore, when the restriction body 20 is placed at the restriction position, the restriction-body axis line O3 and the proximal portion axis line O1 (see Figs. 3A and 3B) are coaxially placed.

As illustrated in Fig. 5, the inner circumferential surface of the screw portion 22 has a restriction-body side tapered annular surface 22a and a restriction-body side straight annular surface 22b. The inner diameter of the restriction-body side tapered annular surface 22a gradually decreases about the restriction-body axis line O3 from one side (that faces the base end Da at the restriction position) thereof toward the other side (that faces the tip end Db at the restriction position) thereof in a direction along the restriction-body axis line O3. The restriction-body side straight annular surface 22b is connected to the other side of the restriction-body side tapered annular surface 22a in a direction along the restriction-body axis line O3 and is precisely annular about the restriction-body axis line O3. The screw portion 22 also has a restriction-body end surface 22c formed in a flat surface facing the one side along the restriction-body axis line O3.

### (Ring body)

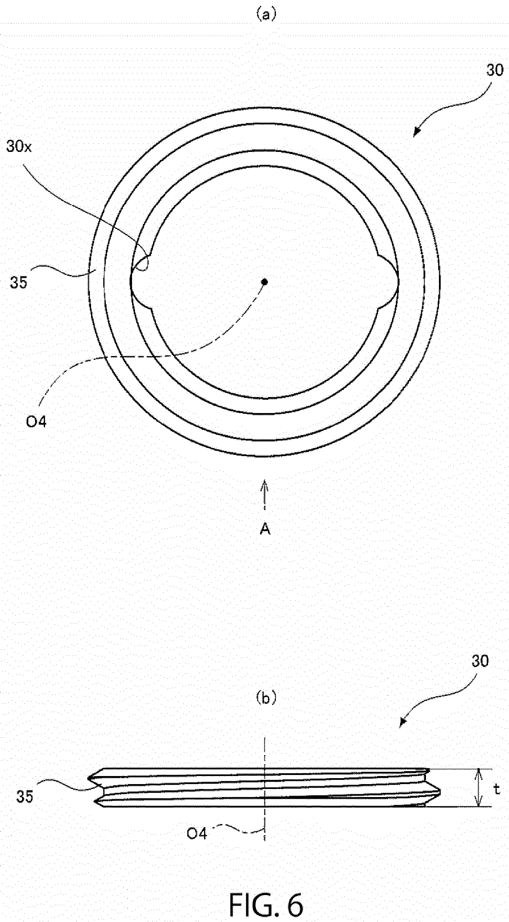

As illustrated in Figs. 6A and 6B, the ring body 30 is precisely annular about a ring-body axis line (first axis line) O4. The ring body 30 is formed of the same material as the restriction body 20. An outer circumferential surface of the ring body 30 has a ring-body side male-thread region 35 formed thereon. This ring-body side male-thread region 35 is formed about the ring-body axis line O4 and can be screwed into the second female-thread region 16 (see Figs. 3A and 3B) in the nail main body 10. When the ring-body side male-thread region 35 is screwed to the second female-thread region 16, the ring-body axis line O4 matches the restriction-body axis line O3 (see Fig. 5).

The length dimension of the ring body 30 in a direction along the ring-body axis line O4 is smaller than an area where the non-threaded region 17 is formed in a direction along the proximal portion axis line O1. In the present embodiment, for example, a thickness dimension t of the ring body 30 in a direction along the ring-body axis line O4 is 13 mm, while an area (formation length) L where the non-threaded region 17 is formed in a direction along the proximal portion axis line O1 (see Figs. 3A and 3B) is 15 mm. The maximum outer diameter, that is, an outer diameter of a thread ridge, of the ring-body side male-thread region 35 is smaller than an inner diameter of the non-threaded region 17. Thus, the ring body 30 can be accommodated in the non-threaded region 17.

The ring body 30 is inserted into the nail main body 10 after the restriction body 20 has been inserted therein, so that the ring body 30 can be accommodated in the non-threaded region 17 with the restriction body 20 already placed at the restriction position (see Fig. 2). When a position at which the ring body 30 is placed in the non-threaded region 17 is referred to as an "in-use position," the ring body 30 is placed at this in-use position when the bone fixation instrument 100 is in use.

Referring back to Fig. 5, an inner circumferential surface of the ring body 30 has a ring-body side tapered annular surface 30a and a ring-body side straight annular surface 30b. An inner diameter of the ring-body side tapered annular surface 30a gradually decreases about the ring-body axis line O4 from one side (that faces the base end Da at the in-use position) thereof toward the other side (that faces the tip end Db at the in-use position) thereof in a direction along the ring-body axis line O4. The ring-body side straight annular surface 30b is connected to the other side of the ring-body side tapered annular surface 30a in a direction along the ring-body axis line O4 and is precisely annular about the ring-body axis line O4. The ring body 30 also has a ring-body end surface 30c formed in a flat surface facing the other side along the ring-body axis line O4.

With the ring-body axis line O4 and the restriction-body axis line O3 coaxially placed, the ring-body end surface 30c of the ring body 30 at the in-use position comes face-to-face with the restriction-body end surface 22c of the restriction body 20 at the restriction position in the longitudinal direction D. With the ring-body axis line O4 and the restriction-body axis line O3 coaxially placed, the entire region of the restriction-body end surface 22c can come face-to-face and contact a part of the ring-body end surface 30c in the longitudinal direction D. More specifically, in the present embodiment, the restriction-body side tapered annular surface 22a that constitutes an inner circumferential edge of the restriction-body end surface 22c is positioned radially outside of the ring-body side straight annular surface 30b that constitutes an inner circumferential edge of the ring-body end surface 30c. In other words, an inner diameter of an inner hole (ring-body inner hole) of the ring-body end surface 30c is smaller than an inner diameter of an inner hole (restriction-body inner hole) of the restriction-body end surface 22c.

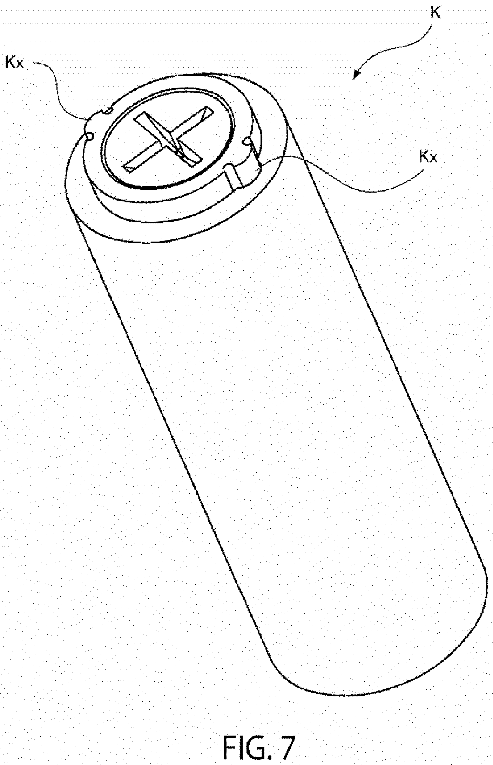

As illustrated in Fig. 6A, the inner circumferential surface of the ring body 30 opens toward one side in a direction along the ring-body axis line O4 and has engagement grooves (engagement portion) 30x formed thereon which are dented radially outwardly. Two of the engagement grooves 30x are formed with equal circumferential distances therebetween (distances of 180 degrees). These engagement grooves 30x are engaged with engagement projections Kx of a dedicated tool K, as illustrated in Fig. 7. The dedicated tool K is rotated with the engagement projections Kx of the dedicated tool K engaging in the engagement grooves 30x, so that the ring body 30 is inserted into the nail main body 10 at the same time as it is screwed into the second female-thread region 16, whereby the ring body 30 can be guided into the non-threaded region 17 of the nail main body 10.

### (End cap)

Referring back to Fig. 2, the end cap 40 is operable to prevent tissue from flowing into the inner holes 11a and 12a of the nail main body 10 (see Figs. 3A and 3B). The end cap 40 is inserted into the inner hole 11a of the proximal portion 11 from one side of the proximal portion axis line O1 and screwed into the second female-thread region 16 of the nail main body 10. The end cap 40 includes a shaft-like region 41 inserted through the inside of the restriction body 20 and the ring body 30, and a screw-engagement region 42 provided on the side of the base end Da of the nail main body 10 to the shaft-like region 41 when the bone fixation instrument 100 is in use, and having a male thread 42a formed on an outer circumferential surface thereof.

When the bone fixation instrument 100 is in use, the shaft-like region 41 is inserted through the restriction body 20 at the restriction position and the ring body 30 at the in-use position with the second female-thread region 16 of the nail main body 10 having the male thread 42a screwed therein. The shaft-like region 41 presses the auxiliary shaft member 3 toward the tip end Db of the nail main body 10 by contacting with an outer circumferential surface of the auxiliary shaft member 3 inserted through both the auxiliary shaft-member insertion hole 10z of the nail main body 10 and the restriction-body through-hole 21x of the restriction body 20.

### (Bone fixation shaft member)

Referring back to Fig. 1, the bone fixation shaft member 2 is provided to the nail main body 10 by being inserted through the shaft-member insertion hole 10y (see Figs. 3A and 3B) of the nail main body 10 when the bone fixation instrument 100 is in use. The bone fixation shaft member 2 is disposed so as to extend from the trochanter part B2 of the femur B towards the bone head B1 thereof. That is, the bone fixation shaft member 2 is disposed inside the femur B in such a manner as to extend upwardly from the lateral side of the body of the patient towards the midline thereof.

The tip end of the bone fixation shaft member 2 has a male thread 2a formed on its outer circumferential surface so that this male thread 2a is screwed into the femur B. In addition, a pressing-force receiving groove 2b is formed on the outer circumferential surface of the bone fixation shaft member 2 extending in an axial direction of the bone fixation shaft member 2 on the side closer to its base end than the male thread 2a. When the bone fixation instrument 100 is in use, the respective tip ends of the paired pressing portions 21b (see Figs. 4A and 4B) of the restriction body 20 are disposed in the pressing-force receiving groove 2b, with the pressing portions 21b being disposed side by side in the axial direction of the bone fixation shaft member 2. The paired pressing portions 21b are operable to press the bone fixation shaft member 2 toward the tip end Db of the nail main body 10, thereby restricting the relative movement and rotation of the bone fixation shaft member 2 to the nail main body 10.

### (Auxiliary shaft member)

The auxiliary shaft member 3 is provided to the nail main body 10 by being inserted through the auxiliary shaft-member insertion hole 10z (see Figs. 3A and 3B) of the nail main body 10 when the bone fixation instrument 100 is in use. When the bone fixation instrument 100 is in use, the auxiliary shaft member 3 is disposed parallel to the bone fixation shaft member 2 above the bone fixation shaft member 2. That is, the auxiliary shaft member 3 is disposed inside the femur B so as to extend from the trochanter part B2 of the femur B towards the bone head B1 thereof. The tip end of the auxiliary shaft member 3 has a male thread 3a formed on its outer circumferential surface so that this male thread 3a is screwed into the femur B. In addition, the outer circumferential surface of the auxiliary shaft member 3 contacts a tip end of the shaft-like region 41 (see Fig. 2) of the end cap 40 on the side closer to its base end than the male thread 3a, and the shaft-like region 41 presses the auxiliary shaft member 3 toward the tip end Db of the nail main body 10, thereby restricting the relative movement and rotation of the auxiliary shaft member 3 to the nail main body 10.

### (Operation and effect)

Before the bone fixation instrument 100 of the present embodiment described above is used to treat a bone fracture, an entry hole is first formed in the upper end of the femur B by using an awl or the like, and then the entry hole is enlarged by using a boring tool such as a drill so that an opening Ba communicating with the medullary cavity of the femur B is formed. Thereafter, after a guide wire (not illustrated) is inserted through the opening Ba, the nail main body 10 is inserted from the opening Ba in such a manner that this guide wire is inserted through the interiors of the nail main body 10, the restriction body 20, and the ring body 30 in that order, with the restriction body 20 and the ring body 30 provided in the interior of the nail main body 10.

Subsequently, a bone hole through which the bone fixation shaft member 2 is inserted and another bone hole through which the auxiliary shaft member 3 is inserted are formed, and the bone fixation shaft member 2 and the auxiliary shaft member 3 are then screwed in. Thereafter, the restriction body 20 is further screwed into the nail main body 10, the bone fixation shaft member 2 is pressed downwardly by the restriction body 20 to be immobilized, and the placement of the bone fixation instrument 100 is completed by finally screwing the end cap 40 into the nail main body 10.

According to the bone fixation instrument 100 of the present embodiment described above, the first female-thread region 15 and the second female-thread region 16 are formed inside the nail main body 10 of the intramedullary nail 1, and the non-threaded region 17 having no female thread is formed between the female-thread regions 15 and 16. Thus, in a state where the ring body 30 is accommodated in the interior of the non-threaded region 17 by being screwed into the second female-thread region 16 and inserted into the interior of the nail main body 10 after the restriction body 20 is inserted into the nail main body 10 and placed at the restriction position where the restriction body 20 is screwed into the first female-thread region 15 only, the ring-body side male-thread region 35 spins inside the non-threaded region 17 without being screwed into the first female-thread region 15 and the second female-thread region 16 even when an external factor causes the ring body 30 to rotate inside the non-threaded region 17.

Therefore, the ring body 30 is prevented from falling off the nail main body 10 and stays inside the non-threaded region 17. Thus, even if the restriction body 20 at the restriction position is about to rotate due to an external factor and move inside the nail main body 10 toward the base end Da (upwardly when the bone fixation instrument 100 is in use) in the longitudinal direction D, the ring body 30 restricts such movement and the falling off of the restriction body 20 and a large displacement of the restriction body 20 from the restriction position can be avoided.

Particularly in the present embodiment, the outer diameter of each thread ridge in the ring-body side male-thread region 35 is smaller than the inner diameter of the non-threaded region 17. Thus, the ring body 30 spins smoothly inside the non-threaded region 17. In this situation, a gap is formed between the ring body 30 and the non-threaded region 17, allowing the ring body 30 to move radially in the non-threaded region 17. As a result, the ring-body axis line O4 of the ring body 30 is shifted from the proximal-portion axis line O1, which serves as the center of the first female-thread region 15 and the second female-thread region 16. This made it possible to reliably avoid falling off of the ring body 30 from the non-threaded region 17.

The inner diameters of the thread ridges in the first female-thread region 15 and the second female-thread region 16 are smaller than the inner diameter of the non-threaded region 17. Thus, bumps are formed between the first female-thread region 15 and the non-threaded region 17 and between the second female-thread region 16 and the non-threaded region 17, and falling of the ring body 30 from the non-threaded region 17 into the female-thread regions 15 and 16 in the longitudinal directions D can be avoided because the ring body 30 comes into contact with the bumps.

An area where the restriction-body side male-thread region 25 is formed in a direction along the restriction-body axis line O3 is larger than an area where the non-threaded region 17 is formed in a direction along the proximal portion axis line O1. Thus, when an attempt is made to screw the restriction body 20 into the nail main body 10 to cause the restriction body 20 to reach a position (for example, the restriction position) at which the restriction-body side male-thread region 25 has been screwed only into the first female-thread region 15 from a position at which the male-thread region 25 has been screwed only into the second female-thread region 16, a state can occur where the restriction-body side male-thread region 25 is screwed into both the second female-thread region 16 and the first female-thread region 15 while the restriction-body side male-thread region 25 is passing through the non-threaded region 17. Therefore, the restriction-body side male-thread region 25 can smoothly pass through the non-threaded region 17, whereby the restriction body 20 can be easily and reliably placed in a predetermined position.

Because the inner circumferential surface of the ring body 30 has the ring-body side tapered annular surface 30a, this ring-body side tapered annular surface 30a is operable to guide the shaft-like region 41 of the end cap 40 when the shaft-like region 41 is inserted into the interior of the ring body 30. Thus, the end cap 40 can be easily placed.

The restriction-body side tapered annular surface 22a that constitutes the inner circumferential edge of the restriction-body end surface 22c is positioned radially outside of the ring-body side straight annular surface 30b that constitutes the inner circumferential edge of the ring-body end surface 30c. Thus, when the ring body 30 is viewed from above with the nail main body 10 placed in the femur B, the inner circumferential edge of the restriction-body end surface 22c is hidden by the ring body 30. This makes it possible to smoothly guide the shaft-like region 41 of the end cap 40 without having the shaft-like region 41 caught between the ring body 30 and the restriction body 20 when the shaft-like region 41 is inserted from the interior of the restriction body 20 toward the interior of the ring body 30.

Besides, the intramedullary nail 1 may be transported in such a manner that the restriction-body end surface 20c and the ring-body end surface 30c are fixed to each other by placing the restriction body 20 and the ring body 30 in the nail main body 10 so that the restriction-body end surface 20c and the ring-body end surface 30c contact each other and internal forces occur in each of the restriction-body end surface 20c and the ring-body end surface 30c. In the present embodiment, the restriction-body end surface 22c contacts a part of the entire region of the ring-body end surface 30c. This makes it possible to minimize the effect of adhesion between the ring body 30 and the restriction body 20, even when the ring body 30 and the restriction body 20 are formed of the same metallic material.

Therefore, even if the restriction body 20 and the ring body 30 adhere to each other, the restriction body 20 can be easily removed from the ring body 30 and the restriction body 20 can be pushed through to the restriction position when the bone fixation instrument 100 is used.

Thus, when the restriction-body end surface 22c contacts the ring-body end surface 30c, the ring body 30 and the restriction body 20 interfere with each other. Therefore, the ring body 30 prevents the restriction body 20 from moving toward the base end Da beyond the ring body 30, thereby avoiding the restriction body 20 from falling off.

The ring body 30 can be rotated only by means of the dedicated tool K. This prevents the ring body 30 from being accidentally removed from the nail main body 10 by a third person.

Obviously, the present invention is not limited to the above embodiments and can be variously changed without departing from the spirit of the present invention.

For example, in the ring body 30, the thickness dimension of the entire ring body 30 in a direction along the ring-body axis line O4 may not necessarily be smaller than an area where the non-threaded region 17 is formed in a direction along the proximal portion axis line O1. It is only necessary that an area where the ring-body side male-thread region 35 is formed in a direction along the ring-body axis line O4 is smaller than an area where the non-threaded region 17 is formed in a direction along the proximal portion axis line O1. In such a case, at least a part of the ring body 30 that is placed inside the non-threaded region 17 is not screwed into either the first female-thread region 15 or the second female-thread region 16 and spins inside the non-threaded region 17, whereby a large relative movement of the restriction body 20 to the nail main body 10 can be avoided.

Furthermore, the ring body 30 may have engagement projections formed thereon instead of the engagement grooves 30x. In such a case, the dedicated tool K needs to have engagement grooves formed thereon instead of the engagement projections Kx.

Furthermore, contrary to the above case, the entire region of the ring-body end surface 30c can come face-to-face and contact a part of the restriction-body end surface 22c in the longitudinal direction D.

An intramedullary nail and a bone fixation instrument of the present invention are capable of improving usability.
- 1: intramedullary nail
- 2: bone fixation shaft member
- 3: auxiliary shaft member
- 10: nail main body
- 10x: upper opening
- 10y: shaft-member insertion hole
- 10z: auxiliary shaft-member insertion hole
- 11: proximal portion
- 11a: inner hole
- 12: distal portion
- 12a: inner hole
- 15: first female-thread region
- 16: second female-thread region
- 17: non-threaded region
- 20: restriction body
- 20c: restriction-body end surface
- 22a: restriction-body side tapered annular surface
- 22c: restriction-body end surface
- 25: restriction-body side male-thread region
- 30: ring body
- 30a: ring-body side tapered annular surface
- 30b: ring-body side straight annular surface
- 30c: ring-body end surface
- 30x: engagement groove
- 35: ring-body side male-thread region
- 100: bone fixation instrument
- B: femur
- B1: bone head
- B2: trochanter portion
- D: longitudinal direction
- Da: base end
- Db: tip end
- K: dedicated tool
- Kx: engagement projection
- O1: proximal-portion axis line (second axis line)
- O2: distal-portion axis line
- O3: restriction-body axis line (third axis line)
- O4: ring-body axis line (first axis line)

## Claims

1. An intramedullary nail (1) that is configured to be inserted into a bone during bone fixation and, together with a bone fixation shaft member (2) inserted through the intramedullary nail, to fix the bone from within the bone, the intramedullary nail comprising:
a nail main body (10) that is cylindrical, has a shaft-member insertion hole (10y) formed at an intermediary position thereof in a longitudinal direction (D) thereof and penetrating an outer circumferential surface thereof, and is configured to have the bone fixation shaft member inserted therethrough, and has a first female-thread region (15) disposed closer to a base end (Da) of the nail main body in the longitudinal direction than the shaft-member insertion hole, a second female-thread region (16) disposed closer to the base end than the first female-thread region, and a non-threaded region (17) disposed between the first female-thread region and the second female-thread region in the longitudinal direction formed on an inner circumferential surface thereof; a restriction body (20) that is cylindrical and has a restriction-body side male-thread region (25) formed on an outer circumferential surface thereof and screwable into the first female-thread region and the second female-thread region, the restriction body being configured to restrict, at a restriction position where the restriction body is screwed into the first female-thread region, relative movement and rotation of the bone fixation shaft member to the nail main body by pressing, toward a tip end (Db) of the nail main body, the bone fixation shaft member disposed in the shaft-member insertion hole; and
a ring body (30) that is annular about a first axis line (O4), has a ring-body side male-thread region (35) formed on an outer circumferential surface thereof and screwable into the second female-thread region, is at least partially disposed within the non-threaded region on the side closer to the base end than the restriction body at the restriction position, at an in-use position,
wherein the first female-thread region and the second female-thread region are formed about a second axis line (O1) extending in the longitudinal direction, and
an area where the ring-body side male-thread region is formed in a direction along the first axis line is smaller than an area where the non-threaded region is formed in a direction along the second axis line.

2. The intramedullary nail according to claim 1, wherein
a thickness dimension (t) of the ring body in a direction along the first axis line is smaller than an area where the non-threaded region is formed in a direction along the second axis line.

3. The intramedullary nail according to claim 1 or 2, wherein
the non-threaded region has a section that is circular about the second axis line, and
an outer diameter of a thread ridge in the ring-body side male-thread region is smaller than an inner diameter of the non-threaded region.

4. The intramedullary nail according to claim 1 or 2, wherein
an inner diameter of a thread ridge in each of the first female-thread region and the second female-thread region is smaller than an inner diameter of the non-threaded region.

5. The intramedullary nail according to claim 1 or 2, wherein
the restriction-body side male-thread region is formed about a third axis line (O3), and
an area where the restriction-body side male-thread region is formed in a direction along the third axis line is larger than an area where the non-threaded region is formed in a direction along the second axis line.

6. The intramedullary nail according to claim 1 or 2, wherein
an inner circumferential surface of the ring body includes a tapered annular surface (22a) formed about the first axis line and having an inner diameter that gradually decreases from one side toward the other side in a direction along the first axis line, the one side being a side facing the base end when the ring body is at the in-use position.

7. The intramedullary nail according to claim 1 or 2, wherein
the restriction body has a restriction-body end surface (22c) that faces the base end at the restriction position, and
the ring body has a ring-body end surface (30c) that faces the tip end at the in-use position and comes face-to-face from the side of the base end with the restriction-body end surface of the restriction body at the restriction position.

8. The intramedullary nail according to claim 7, wherein
the restriction body is cylindrical about a third axis line (O3), and
an inner circumferential edge of the restriction-body end surface is disposed radially outside of an inner circumferential edge of the ring-body end surface when the first axis line and the third axis line are coaxially placed.

9. The intramedullary nail according to claim 7, wherein
the restriction-body end surface has a restriction-body inner hole formed inside thereof that is circular about a third axis line (O3) extending in the longitudinal direction when the restriction body is at the restriction position, and
the ring-body end surface has a ring-body inner hole formed inside thereof that is circular about the first axis line and has a smaller diameter than the restriction-body inner hole.

10. The intramedullary nail according to claim 7, wherein
the restriction body and the ring body are formed of the same metallic material, and
the entire region of one of the restriction-body end surface and the ring-body end surface is configured to come face-to-face with a part of the entire region of the other thereof in the longitudinal direction.

11. The intramedullary nail according to claim 1 or 2, wherein
the ring body has an engagement portion (30x) formed on an inner circumferential surface thereof,
the engagement portion being dented radially outwardly or projected radially inwardly, and
the ring body is rotatable about the first axis line by use of a tool (K) with the engagement portion engaged with an engagement receiving portion (Kx) of the tool.

12. A bone fixation instrument comprising:
the intramedullary nail according to claim 1 or 2; and
the bone fixation shaft member configured to be inserted through the shaft-member insertion hole in the intramedullary nail.

## Patentansprüche

1. Marknagel, der während einer Knochenfixierung in einen Knochen eingeführt wird und zusammen mit einem Knochenfixierungsschaftelement, das durch den Marknagel eingeführt wird, den Knochen von innerhalb des Knochens fixiert, wobei der Marknagel Folgendes umfasst:
einen Nagelhauptkörper, der zylindrisch ist, ein Schaftelement-Einführloch aufweist, das an einer Zwischenposition davon in einer Längsrichtung davon ausgebildet ist und eine Außenumfangsfläche davon durchdringt, und konfiguriert ist, um das Knochenfixierungsschaftelement dadurch eingeführt zu haben, und einen ersten Innengewindebereich, der näher an einem Basisende des Nagelhauptkörpers in der Längsrichtung als das Schaftelement-Einführloch angeordnet ist, einen zweiten Innengewindebereich, der näher an dem Basisende als der erste Innengewindebereich angeordnet ist, und einen Nicht-Gewindebereich, der zwischen dem ersten Innengewindebereich und dem zweiten Innengewindebereich in der Längsrichtung angeordnet ist, der auf einer Innenumfangsfläche davon ausgebildet ist, aufweist;
einen Beschränkungskörper, der zylindrisch ist und einen Beschränkungskörperseiten-Außengewindebereich aufweist, der auf einer Außenumfangsfläche davon ausgebildet ist und in den ersten Innengewindebereich und den zweiten Innengewindebereich schraubbar ist, wobei der Beschränkungskörper konfiguriert ist, um an einer Beschränkungsposition, an der der Beschränkungskörper in den ersten Innengewindebereich geschraubt ist, eine relative Bewegung und Drehung des Knochenfixierungsschaftelements zu dem Nagelhauptkörper durch Drücken, in Richtung des Spitzenendes, des Knochenfixierungsschaftelements, das in dem Schaftelement-Einführloch angeordnet ist, zu beschränken; und
einen Ringkörper, der ringförmig um eine erste Achsenlinie ist, einen Ringkörperseiten-Außengewindebereich aufweist, der auf einer Außenumfangsfläche davon ausgebildet ist und in den zweiten Innengewindebereich schraubbar ist, zumindest teilweise innerhalb des Nicht-Gewindebereichs auf der Seite näher an dem Basisende als der Beschränkungskörper an der Beschränkungsposition an einer Gebrauchsposition angeordnet ist,
wobei der erste Innengewindebereich und der zweite Innengewindebereich um eine zweite Achsenlinie, die sich in der Längsrichtung erstreckt, ausgebildet sind, und
eine Fläche, an der der Ringkörperseiten-Außengewindebereich in einer Richtung entlang der ersten Achsenlinie ausgebildet ist, kleiner ist als eine Fläche, an der der Nicht-Gewindebereich in einer Richtung entlang der zweiten Achsenlinie ausgebildet ist.

2. Marknagel nach Anspruch 1, wobei
eine Dickenabmessung des Ringkörpers in einer Richtung entlang der ersten Achsenlinie kleiner ist als eine Fläche, an der der Nicht-Gewindebereich in einer Richtung entlang der zweiten Achsenlinie ausgebildet ist.

3. Marknagel nach Anspruch 1 oder 2, wobei
der Nicht-Gewindebereich einen Abschnitt aufweist, der kreisförmig um die zweite Achsenlinie ist, und
ein Außendurchmesser eines Gewindekamms in dem Ringkörperseiten-Außengewindebereich kleiner ist als ein Innendurchmesser des Nicht-Gewindebereichs.

4. Marknagel nach Anspruch 1 oder 2, wobei
ein Innendurchmesser eines Gewindekamms in jedem von dem ersten Innengewindebereich und dem zweiten Innengewindebereich kleiner ist als ein Innendurchmesser des Nicht-Gewindebereichs.

5. Marknagel nach Anspruch 1 oder 2, wobei
der Beschränkungskörperseiten-Außengewindebereich um die dritte Achsenlinie ausgebildet ist, und
eine Fläche, an der der Beschränkungskörperseiten-Außengewindebereich in einer Richtung entlang der ersten Achsenlinie ausgebildet ist, größer ist als eine Fläche, an der der Nicht-Gewindebereich in einer Richtung entlang der zweiten Achsenlinie ausgebildet ist.

6. Marknagel nach Anspruch 1 oder 2, wobei
eine Innenumfangsfläche des Ringkörpers eine verjüngte ringförmige Fläche beinhaltet, die um die erste Achsenlinie ausgebildet ist und einen Innendurchmesser aufweist, der allmählich von einer Seite zu der anderen Seite in einer Richtung entlang der ersten Achsenlinie abnimmt,
wobei die eine Seite eine Seite ist, die dem Basisende zugewandt ist, wenn sich der Ringkörper an der Gebrauchsposition befindet.

7. Marknagel nach Anspruch 1 oder 2, wobei
der Beschränkungskörper eine Beschränkungskörperendfläche aufweist, die dem Basisende an der Beschränkungsposition zugewandt ist, und
der Ringkörper eine Ringkörperendfläche aufweist, die dem Spitzenende an der Gebrauchsposition zugewandt ist und von der Seite des Basisendes mit der Beschränkungskörperendfläche des Beschränkungskörpers an der Beschränkungsposition einander zugewandt ist.

8. Marknagel nach Anspruch 7, wobei
der Beschränkungskörper zylindrisch um eine dritte Achsenlinie ist, und
eine Innenumfangskante der Beschränkungskörperendfläche radial außerhalb einer Innenumfangskante der Ringkörperendfläche angeordnet ist, wenn die erste Achsenlinie und die dritte Achsenlinie koaxial platziert sind.

9. Marknagel nach Anspruch 7, wobei
die Beschränkungskörperendfläche ein Beschränkungskörper-Innenloch aufweist, das darin ausgebildet ist, das kreisförmig um eine dritte Achsenlinie ist, die sich in der Längsrichtung erstreckt, wenn der Beschränkungskörper an der Beschränkungsposition ist, und
die Ringkörperendfläche ein Ringkörper-Innenloch aufweist, das darin ausgebildet ist, das kreisförmig um die erste Achsenlinie ist und einen kleineren Durchmesser als das Beschränkungskörper-Innenloch aufweist.

10. Marknagel nach Anspruch 7, wobei
der Beschränkungskörper und der Ringkörper aus dem gleichen metallischen Material ausgebildet sind, und
der gesamte Bereich von einem von der Beschränkungskörperendfläche und der Ringkörperendfläche konfiguriert ist, um mit einem Teil des gesamten Bereichs des anderen davon in der Längsrichtung einander zugewandt zu sein.

11. Marknagel nach Anspruch 1 oder 2, wobei
der Ringkörper einen Eingriffsabschnitt aufweist, der auf einer Innenumfangsfläche davon ausgebildet ist, wobei der Eingriffsabschnitt radial nach außen eingedrückt ist oder radial nach innen vorsteht, und
der Ringkörper um die erste Achsenlinie unter Verwendung eines Werkzeugs drehbar ist, wobei der Eingriffsabschnitt mit einem Eingriffsaufnahmeabschnitt des Werkzeugs in Eingriff steht.

12. Knochenfixierungsinstrument, das Folgendes umfasst:
den Marknagel nach Anspruch 1 oder 2; und
ein Knochenfixierungsschaftelement, das konfiguriert ist, um durch das Schaftelementeinführloch in dem Marknagel eingeführt zu werden.

## Revendications

1. Clou intramédullaire qui est inséré dans un os pendant la fixation osseuse et, conjointement avec un élément d'arbre de fixation osseuse inséré à travers le clou intramédullaire, fixe l'os depuis l'intérieur de l'os, le clou intramédullaire comprenant :
un corps principal de clou qui est cylindrique, a un trou d'insertion d'élément d'arbre formé à une position intermédiaire de celui-ci dans une direction longitudinale de celui-ci et pénétrant une surface circonférentielle externe de celui-ci, et est configuré pour avoir l'élément d'arbre de fixation osseuse inséré à travers celui-ci, et a une première région de filetage femelle disposée plus près d'une extrémité de base du corps principal de clou dans la direction longitudinale que le trou d'insertion d'élément d'arbre, une seconde région de filetage femelle disposée plus près de l'extrémité de base que la première région de filetage femelle, et une région non filetée disposée entre la première région de filetage femelle et la seconde région de filetage femelle dans la direction longitudinale formée sur une surface circonférentielle interne de celle-ci ;
un corps de restriction qui est cylindrique et a une région de filetage mâle côté corps de restriction formée sur une surface circonférentielle externe de celui-ci et vissable dans la première région de filetage femelle et la seconde région de filetage femelle, le corps de restriction étant configuré pour restreindre, à une position de restriction où le corps de restriction est vissé dans la première région de filetage femelle, le mouvement relatif et la rotation de l'élément d'arbre de fixation osseuse par rapport au corps principal de clou en pressant, vers l'extrémité de pointe, l'élément d'arbre de fixation osseuse disposé dans le trou d'insertion d'élément d'arbre ; et
un corps annulaire qui est annulaire autour d'une première ligne d'axe, a une région de filetage mâle côté corps annulaire formée sur une surface circonférentielle externe de celui-ci et vissable dans la seconde région de filetage femelle, est au moins partiellement disposé à l'intérieur de la région non filetée sur le côté plus près de l'extrémité de base que le corps de restriction à la position de restriction, à une position d'utilisation,
dans lequel la première région de filetage femelle et la seconde région de filetage femelle sont formées autour d'une seconde ligne d'axe s'étendant dans la direction longitudinale, et
une zone où la région de filetage mâle côté corps annulaire est formée dans une direction le long de la première ligne d'axe est plus petite qu'une zone où la région non filetée est formée dans une direction le long de la seconde ligne d'axe.

2. Clou intramédullaire selon la revendication 1, dans lequel
une dimension d'épaisseur du corps annulaire dans une direction le long de la première ligne d'axe est plus petite qu'une zone où la région non filetée est formée dans une direction le long de la seconde ligne d'axe.

3. Clou intramédullaire selon la revendication 1 ou 2, dans lequel
la région non filetée a une section qui est circulaire autour de la seconde ligne d'axe, et
un diamètre externe d'une crête de filetage dans la région de filetage mâle côté corps annulaire est plus petit qu'un diamètre interne de la région non filetée.

4. Clou intramédullaire selon la revendication 1 ou 2, dans lequel
un diamètre interne d'une crête de filetage dans chacune de la première région de filetage femelle et de la seconde région de filetage femelle est plus petit qu'un diamètre interne de la région non filetée.

5. Clou intramédullaire selon la revendication 1 ou 2, dans lequel
la région de filetage mâle côté corps de restriction est formée autour de la troisième ligne d'axe, et
une zone où la région de filetage mâle côté corps de restriction est formée dans une direction le long de la première ligne d'axe est plus grande qu'une zone où la région non filetée est formée dans une direction le long de la seconde ligne d'axe.

6. Clou intramédullaire selon la revendication 1 ou 2, dans lequel
une surface circonférentielle interne du corps annulaire comprend une surface annulaire effilée formée autour de la première ligne d'axe et ayant un diamètre interne qui diminue progressivement d'un côté vers l'autre côté dans une direction le long de la première ligne d'axe, le côté étant un côté faisant face à l'extrémité de base lorsque le corps annulaire est à la position d'utilisation.

7. Clou intramédullaire selon la revendication 1 ou 2, dans lequel
le corps de restriction a une surface d'extrémité de corps de restriction qui fait face à l'extrémité de base à la position de restriction, et
le corps annulaire a une surface d'extrémité de corps annulaire qui fait face à l'extrémité de pointe à la position d'utilisation et vient face à face à partir du côté de l'extrémité de base avec la surface d'extrémité de corps de restriction du corps de restriction à la position de restriction.

8. Clou intramédullaire selon la revendication 7, dans lequel
le corps de restriction est cylindrique autour d'une troisième ligne d'axe, et
un bord circonférentiel interne de la surface d'extrémité de corps de restriction est disposé radialement à l'extérieur d'un bord circonférentiel interne de la surface d'extrémité de corps annulaire lorsque la première ligne d'axe et la troisième ligne d'axe sont placées coaxialement.

9. Clou intramédullaire selon la revendication 7, dans lequel
la surface d'extrémité de corps de restriction a un trou interne de corps de restriction formé à l'intérieur de celle-ci qui est circulaire autour d'une troisième ligne d'axe s'étendant dans la direction longitudinale lorsque le corps de restriction est à la position de restriction, et
la surface d'extrémité de corps annulaire a un trou interne de corps annulaire formé à l'intérieur de celle-ci qui est circulaire autour de la première ligne d'axe et a un diamètre plus petit que le trou interne de corps de restriction.

10. Clou intramédullaire selon la revendication 7, dans lequel
le corps de restriction et le corps annulaire sont formés du même matériau métallique, et
la région entière de l'une de la surface d'extrémité de corps de restriction et de la surface d'extrémité de corps annulaire est configurée pour venir face à face avec une partie de la région entière de l'autre de celles-ci dans la direction longitudinale.

11. Clou intramédullaire selon la revendication 1 ou 2, dans lequel
le corps annulaire a une partie de mise en prise formée sur une surface circonférentielle interne de celui-ci, la partie de mise en prise étant dentée radialement vers l'extérieur ou faisant saillie radialement vers l'intérieur, et
le corps annulaire peut tourner autour de la première ligne d'axe en utilisant un outil avec la partie de mise en prise mise en prise avec une partie de réception de mise en prise de l'outil.

12. Instrument de fixation osseuse comprenant :
le clou intramédullaire selon la revendication 1 ou 2 ; et
un élément d'arbre de fixation osseuse configuré pour être inséré à travers le trou d'insertion d'élément d'arbre dans le clou intramédullaire.
